Europäisches Patentamt

European Patent Office

Office européen des brevets

(11) Publication number: **0 584 376 A1**

## EUROPEAN PATENT APPLICATION
### published in accordance with Art. 158(3) EPC

(21) Application number: 93905638.8

(22) Date of filing: 12.03.93

(86) International application number: PCT/JP93/00304

(87) International publication number: WO 93/17652 (16.09.93 93/22)

(51) Int. Cl.5: **A61K 6/00, A61K 6/083, C08F 2/44**

(30) Priority: 13.03.92 JP 55509/92
13.03.92 JP 55511/92

(43) Date of publication of application:
02.03.94 Bulletin 94/09

(84) Designated Contracting States:
**DE FR GB**

(71) Applicant: **MITSUBISHI RAYON CO., LTD.**
**3-19, Kyobashi 2-chome**
**Chuo-Ku**
**Tokyo 104(JP)**

(72) Inventor: **YAMAMOTO, Naoki, Central**
**Research Laboratories**
**Mitsubishi Rayon Co., Ltd.,**
**20-1, Miyukicho**
**Otake-shi, Hiroshima 739-06(JP)**
Inventor: **IGE, Hitoshi, Central Research**
**Laboratories**
**Mitsubishi Rayon Co., Ltd.,**
**20-1, Miyukicho**
**Otake-shi, Hiroshima 739-06(JP)**
Inventor: **MAKINO, Takayuki, Central**
**Research Laboratories**

**Mitsubishi Rayon Co., Ltd.,**
**20-1, Miyukicho**
**Otake-shi, Hiroshima 739-06(JP)**
Inventor: **YAMASAKI, Hiroko, Central**
**Research Laboratories**
**Mitsubishi Rayon Co., Ltd.,**
**20-1, Miyukicho**
**Otake-shi, Hiroshima 739-06(JP)**
Inventor: **TAYAMA, Suehiro, Otake Plants**
**Mitsubishi Rayon Co. Ltd.,**
**20-1, Miyukicho**
**Otake-shi, Hiroshima 739-06(JP)**
Inventor: **TORITANI, Akihiro, Otake Plants**
**Mitsubishi Rayon Co., Ltd.,**
**20-1, Miyukicho**
**Otake-shi, Hiroshima 739-06(JP)**
Inventor: **MUKAI, Nobuhiro, Toyama Plants**
**Mitsubishi Rayon Co. Ltd.,**
**3, Kaigandori**
**Toyama-shi, Toyama 931(JP)**

(74) Representative: **TER MEER - MÜLLER -**
**STEINMEISTER & PARTNER**
**Mauerkircherstrasse 45**
**D-81679 München (DE)**

(54) **SPHERICAL PARTICLE AS DENTAL PLATE MATERIAL, PRODUCTION THEREOF, AND DENTAL PLATE RESIN COMPOSITION.**

(57) Spherical particles (A) as dental plate material comprising a graft copolymer (A-1) of a cross-linked rubbery (co)polymer with a rigid resin component and a methyl methacrylate (co)polymer (A-2). The particles (A) are compounded with a monofunctional methacrylic ester (B), or a mixture thereof with a multifunctional methacrylic ester, and a polymerization initiator (C) to give a dental plate resin composition which is excellent in the workability in molding and provides a molded article having excellent impact resistance and being very useful as a dental material.

Rank Xerox (UK) Business Services
(3.10/3.09/3.3.4)

## Technical Field

This invention relates to spherical particles for use in denture base materials having excellent impact resistance and workability, a process for preparing the same, and resin compositions for the formation of denture bases.

## Background Art

At present, acrylic resin denture base materials obtained by blending a monomer such as methyl methacrylate with a polymer formed from such a monomer and polymerising the blend by the application of heat are widely used because of their excellent transparency, surface gloss and moldability. However, such acrylic resin denture base materials are insufficient in mechanical strength properties such as impact resistance and, therefore, have the disadvantage that thin-wall portions of the denture may be damaged by occlusion. As an attempt to overcome this disadvantage, it has been proposed in Japanese Patent Laid-Open No. 275509/'89 that a powder of an elastomer such as urethane rubber, silicone rubber, polybutadiene, butyl rubber, nitrile rubber, natural rubber, chloroprene rubber or fluororubber is blended with a methyl methacrylate monomer or an unsaturated urethane monomer and the resulting composition is polymerized to obtain a dental material. Moreover, denture bases formed of thermoplastic resins such as polyethersulfones, polysulfones, polycarbonates and aromatic polyester copolymers have also been proposed in Japanese Patent Laid-Open Nos. 49849/'87 and 341/'87.

However, the denture base materials obtained according to the process described in Japanese Patent Laid-Open No. 275509/'89 do not have good compatibility between methyl methacrylate and the elastomer powder. Moreover, since methyl methacrylate and the elastomer powder are simply blended together, the elastomer powder is not dispersed well and hence fails to produce the desired impact resistance. Furthermore, since the particle shape of the elastomer powder used is irregular, the wettability of the elastomer powder by methyl methacrylate is so poor that it is difficult to blend the elastomer powder with methyl methacrylate and thereby prepare a composition.

On the other hand, the thermoplastic resins (such as polyethersulfones, polysulfones, polycarbonates and aromatic polyester copolymers) described in Japanese Patent Laid-Open Nos. 49849/'87 and 341/'87 must be molded at high temperatures. Thus, they have the disadvantage of being poor in moldability and undergoing dimensional changes especially due to thermal shrinkage.

In addition, European Patent No. 411512 discloses dental compositions comprising a (meth)acrylic ester monomer, a polymer thereof, and an acrylic rubber graft copolymer compatible therewith. Also in this case, the acrylic rubber graft copolymer is not dispersed well and hence fails to produce sufficient impact resistance. Moreover, since the acrylic rubber graft copolymer has significant oil absorption properties, it absorbs the monomer in the preparation of compositions. Thus, these components are not easily blended at an ordinary powder/liquid ratio, exhibiting very poor workability. Although the problem of poor workability can be solved by increasing the proportion of the monomer in the composition, this causes an increase in polymerization shrinkage at the time of curing and thereby makes it impossible to obtain precision moldings.

Accordingly, there is a strong demand for a material which retains the properties possessed by the above-described acrylic resin denture base materials and, in addition, exhibits high impact resistance.

In view of these circumstances, the present inventors have made an intensive study and have found that, if spherical particles comprising polymethyl methacrylate or a methyl methacrylate-based polymer and a rubber graft copolymer having a specific structure are used in denture base materials, the resulting acrylic resin denture base materials not only have the excellent properties inherent therein, but also exhibit excellent impact resistance due to the good compatibility and uniform dispersibility of the spherical particles containing a rubber component with the (meth)acrylic ester monomer, as well as excellent workability at the time of blending with the monomer due to the incorporation of the rubber component in the spherical particles. The present invention has been completed on the basis of this finding.

## Disclosure of the Invention

According to one aspect of the present invention, there are provided spherical particles (A) for use in denture base materials consisting of a graft copolymer (A-1) formed by the graft polymerization of a rigid resin component onto a crosslinked rubbery (co)polymer, and a methyl methacrylate (co)polymer (A-2).

According to another aspect of the present invention, there is provided a process for preparing the above-described spherical particles (A) by dispersing the above-described graft copolymer (A-1) in methyl methacrylate, a monomer mixture containing methyl methacrylate, or a partial polymer thereof, and subjecting the resulting mixture to suspension polymerization, or by dissolving the methyl methacrylate (co)polymer (A-2) and the graft copolymer (A-1) in a solvent, dispersing the resulting solution in an aqueous or gaseous phase, and removing the solvent.

According to still another aspect of the present invention, there is provided a resin composition for the formation of denture bases comprising the above-described spherical particles (A), a component (B) comprising one or more monofunctional methacrylic esters or a mixture of one or more monofunctional methacrylic esters and one or more multifunctional methacrylic esters, and a polymerization initiator (C).

## Best Mode for Carrying Out the Invention

The crosslinked rubbery (co)polymers which can be used in the present invention include butadiene (co)polymers, polyalkyl acrylate rubber copolymers, and composite rubbers formed by polymerizing a polyalkyl acrylate rubber copolymer in the presence of a butadiene polymer.

Useful butadiene (co)polymers include polymers formed from 5% by weight or more of 1,3-butadiene and 95% by weight or less of one or more monomers copolymerizable with 1,3-butadiene.

Monomers copolymerizable with 1,3-butadiene include styrene, acrylonitrile, alkyl acrylates having an alkyl group of 2 to 8 carbon atoms, and the like. If desired, butadiene copolymers may additionally contain multifunctional monomers such as divinylbenzene, ethylene glycol dimethacrylate, butylene glycol dimethacrylate, trimethylolpropane triacrylate and pentaerythritol tetraacrylate. A preferred monomer copolymerizable with butadiene is butyl acrylate.

Useful polyalkyl acrylate rubber copolymers include copolymers containing, as essential components, one or more alkyl acrylates having an alkyl group of 2 to 8 carbon atoms and one or more multifunctional monomers. If desired, copolymers formed from mixtures of the above-described essential components and monomers copolymerizable therewith can also be used. Such copolymerizable monomers include styrene, styrene derivatives such as α-methylstyrene and vinyltoluene, acrylonitrile, methyl methacrylate and the like. Among others, styrene and mixtures of styrene and styrene derivatives are preferred.

Alkyl acrylates having an alkyl group of 2 to 8 carbon atoms include, for example, ethyl acrylate, n-butyl acrylate and 2-ethylhexyl acrylate. Among them, butyl acrylate is preferred.

Useful multifunctional monomers include ethylene glycol di(meth)acrylate, 1,3-butylene glycol dimethacrylate, tetraethylene glycol dimethacrylate, divinylbenzene, trimethylolpropane triacrylate, pentaerythritol tetraacrylate, allyl (meth)acrylate and the like.

Although no particular limitation is placed on the proportions of the components constituting the polyalkyl acrylate rubber copolymers, they preferably comprise 69.9 to 99.9% by weight of an alkyl acrylate, 0.1 to 10% by weight of a multifunctional monomer and 0 to 30% by weight of a monomer copolymerizable therewith. More preferably, they comprise 69.9 to 89.9% by weight of an alkyl acrylate, 0.1 to 10% by weight of a multifunctional monomer and 10 to 30% by weight of a monomer copolymerizable therewith.

The crosslinked rubbery (co)polymer used in the present invention preferably has a particle diameter (number-average particle diameter) of 0.1 to 1 $\mu$m. Crosslinked rubbery (co)polymers having such a particle diameter are preferably prepared by emulsion polymerization. If the particle diameter is less than 0.1 $\mu$m, the crosslinked rubbery (co)polymer tends to be scarcely effective in improving impact strength, while if it is greater than 1 $\mu$m, the crosslinked rubbery (co)polymer tends to produce a poor surface appearance.

In preparing a rubber having a particle diameter of 0.1 $\mu$m or greater, this can be done by preparing a latex of a crosslinked rubbery (co)polymer having a particle diameter of less than 0.1 $\mu$m and then enlarging this latex to an average particle diameter of 0.1 to 1 $\mu$m.

Where it is desired to use a composite rubber as the rubber and increase the particle diameter of the composite rubber, this can be done by enlarging the particles of a butadiene rubber and then polymerizing a polyalkyl acrylate rubber in the presence thereof, or by preparing a composite rubber and then enlarging its particles. Although no particular limitation is placed on the method of particle enlargement, it is preferable to employ a method in which a latex of a polyacrylic ester copolymer containing a component derived from an unsaturated acid is added to a latex of the crosslinked rubbery (co)polymer.

As monomers for the formation of the rigid resin component which is grafted onto the crosslinked rubbery (co)polymer in the present invention, there may be used styrene, methyl methacrylate, mixtures thereof, and mixtures of these monomers and monomers copolymerizable therewith. Useful copolymerizable monomers include acrylic esters such as methyl acrylate and ethyl acrylate; methacrylic esters such as phenyl methacrylate, benzyl methacrylate and isopropyl methacrylate; acrylonitrile; and mixtures thereof. Among these monomers, methyl methacrylate alone or a mixture of 90% by weight or more of methyl methacrylate and 10% by weight or less of styrene or a copolymerizable monomer as described above is preferably used.

The graft copolymer, which comprises the crosslinked rubbery (co)polymer having a rigid resin component grafted thereonto, can be prepared by adding 10 to 1,000 parts by weight (preferably 10 to 400 parts by weight) of a monomer for the formation of the rigid resin component to 100 parts by weight, on a solid basis, of a latex of the crosslinked rubbery (co)polymer, allowing the monomer to penetrate into the crosslinked rubbery (co)polymer, and then effecting graft polymerisation with the aid of a conventional polymerization initiator.

The graft copolymer used in the present invention can be a (partial) coagulum. A (partial) coagulum of the graft copolymer can be obtained by adding a coagulant to a latex of the graft copolymer. For this purpose, acids and water-soluble inorganic salts can be used as coagulants. Useful acids include mineral acids such as sulfuric acid and hydrochloric acid, and organic acids having a dissociation constant of not less than $10^6$ moles per liter, such as acetic acid, benzoic acid, salicylic acid, formic acid and tartaric acid. Useful inorganic salts include sulfates (such as magnesium sulfate and sodium sulfate), chlorides and acetates.

In the present invention, the spherical particles (A) consist of the graft copolymer (A-1) and the methyl methacrylate (co)polymer (A-2). In these spherical particles (A), the graft copolymer (A-1) is preferably contained 3 to 60% by weight, more preferably 5 to 25% by weight.

The methyl methacrylate (co)polymer (A-2) preferably comprises a polymer formed from methyl methacrylate alone or a mixture of 50% by weight or more of methyl methacrylate and 50% by weight or less of at least one monomer copolymerizable therewith. More preferably, it is formed from methyl methacrylate alone or a mixture of 90% by weight or more of methyl methacrylate and 10% by weight or less of a copolymerizable monomer.

The copolymerizable monomers which can be used in combination with methyl methacrylate monomer include methacrylic esters such as ethyl methacrylate, isopropyl methacrylate, butyl methacrylate, 2-ethylhexyl methacrylate, phenyl methacrylate and benzyl methacrylate; acrylic esters such as methyl acrylate, ethyl acrylate, butyl acrylate and 2-ethylhexyl acrylate; unsaturated carboxylic acids such as acrylic acid, methacrylic acid, maleic acid and itaconic acid; acid anhydrides such as maleic anhydride and itaconic anhydride; maleimide derivatives such as N-phenylmaleimide, N-cyclohexylmaleimide and N-t-butylmaleimide; hydroxyl-containing monomers such as 2-hydroxyethyl acrylate, 2-hydroxypropyl acrylate, 2-hydroxyethyl methacrylate and 2-hydroxypropyl methacrylate; nitrogen-containing monomers such as acrylamide, methacrylamide, acrylonitrile, methacrylonitrile, diacetone acrylamide and dimethylaminoethyl methacrylate; epoxy-containing monomers such as allyl glycidyl ether, glycidyl acrylate and glycidyl methacrylate; styrenic monomers such as styrene and $\alpha$-methylstyrene; crosslinking agents such as ethylene glycol diacrylate, allyl acrylate, ethylene glycol dimethacrylate, allyl methacrylate, divinylbenzene and trimethylolpropane triacrylate. Especially where denture base resins having high transparency are desired, copolymerizable monomers such as phenyl methacrylate, benzyl methacrylate and isopropyl methacrylate are preferably used in an amount of 10% or less for the purpose of compensating the slight difference in refractive index between the methacrylate resin and the rubber phase.

The spherical particles (A) can be prepared by suspension polymerization using a conventional suspension stabilizer such as polyvinyl alcohol. Specifically, there may employed a process in which a mixed solution composed of methyl methacrylate, a mixture of methyl methacrylate and at least one monomer copolymerizable therewith, or a partial polymer thereof (incompletely polymerized monomer solution), and the above-described graft copolymer is added dropwise to an aqueous solution containing a suspension stabilizer and subjected to suspension polymerization. It is important that the mixed solution is prepared by a means which can achieve high dispersion of the graft copolymer (for example, agitation in a homomixer).

As the suspension stabilizer, there may used any of conventional inorganic and organic dispersing agents. Useful inorganic dispersing agents include magnesium carbonate, calcium tertiary phosphate and the like. Useful organic dispersing agents include starch, gelatin, acrylamide, polyvinyl alcohol, polyacrylic acid and its salts (for example, methyl methacrylate-potassium acrylate copolymer), cellulose, methyl cellulose, hydroxymethyl cellulose, hydroxyethyl cellulose, polyalkylene oxide, polyvinyl pyrrolidone, polyvinyl imidazole and the like. In addition, conventional emulsifying agents such as alkylbenzenesulfonates and fatty acid salts can also be used as low-molecular-weight dispersing agents. Among others, polyvinyl alcohol, polyacrylic acid and its salts are preferred. Especially preferred is polyvinyl alcohol.

In the suspension polymerization, an organic peroxide or an azo compound can be used as the polymerization initiator. Specifically, benzoyl peroxide, lauroyl peroxide, azobisisobutyronitrile or the like can be used.

The diameter of the spherical particles (A) thus obtained can be arbitrarily determined according to the type and amount of dispersing agent used and the shearing force of the agitation. However, the average particle diameters is preferably in the range of 1 to 500 $\mu$m. From the viewpoint of workability, average particle diameters of 50 to 200 $\mu$m are most suitable.

The spherical particles (A) can also be prepared by dissolving the methyl methacrylate (co)polymer and the graft copolymer in a solvent, dispersing the resulting solution in an aqueous or gaseous phase, and then removing the solvent. In this case, the methyl methacrylate (co)polymer and the graft copolymer may be simply mixed in the solution. Alternatively, it is also possible to use a blend obtained by melt-blending the methyl methacrylate (co)polymer and the graft copolymer, a solid obtained by dispersing the graft copolymer in a monomer mixture containing methyl methacrylate and polymerizing this mixture, or a ground powder thereof. In any event, it is important to use a means which can achieve high dispersion of the graft copolymer.

For this purpose, there may be any solvent that can dissolve the methyl methacrylate (co)polymer. Among others, chloroform, dichloromethane, toluene, ethyl acetate and methyl methacrylate are preferred.

No particular limitation is placed on the method by which the solution formed by dissolving the methyl methacrylate (co)polymer and the graft copolymer in a solvent is dispersed in an aqueous phase. For example, the solution can be dispersed in an aqueous phase with the aid of a suspension stabilizer usually used in suspension polymerization, such as polyvinyl alcohol. Although no particular limitation is placed on the method for removing the solvent from the solution dispersed in the aqueous phase, it is possible to evaporate the solvent, for example, by warming the dispersion to 30-90 °C with stirring or exposing the dispersion to reduced pressure.

In the case where the solution formed by dissolving the methyl methacrylate (co)polymer and the graft copolymer in a solvent is dispersed in a gaseous phase and the solvent is then removed, there may be employed, for example, a process using spray drying. The conditions of spray drying (such as temperature and flow rate) can be determined according to the type of solvent used and the temperature used, which makes it possible to obtain spherical particles (A) having any desired particle diameter (in the range of 1 to 500 $\mu$m).

The monofunctional methacrylic esters which can be used as component (B) in the present invention include methyl methacrylate, ethyl methacrylate, propyl methacrylate, butyl methacrylate, hydroxyethyl methacrylate, benzyl methacrylate, methoxyethyl methacrylate, glycidyl methacrylate, tetrahydrofurfuryl methacrylate, monoesters formed from trimellitic acid and hydroxyethyl methacrylate, and the like. Among others, methyl methacrylate is preferred.

Useful multifunctional methacrylic esters include ethylene glycol dimethacrylate, diethylene glycol dimethacrylate, triethylene glycol dimethacrylate, 1,3-butanediol dimethacrylate, 1,4-butanediol dimethacrylate, 1,6-hexanediol dimethacrylate, bisphenol A dimethacrylate, trimethylolpropane dimethacrylate, pentaerythritol tetramethacrylate, dipentaerythritol tetramethacrylate and the like. Where a mixture of monofunctional and multifunctional methacrylic esters is used, it is preferable to use methyl methacrylate as the principal ingredient in an amount of not less than 90% by weight.

As the polymerization initiator (C), there can be used any of commonly used polymerization initiators including organic peroxides, azo compounds, photopolymerization initiators and the like. However, organic peroxides are preferably used. Useful organic peroxides include benzoyl peroxide, 4,4-dichlorobenzoyl peroxide, dilauroyl peroxide and the like. Among others, benzoyl peroxide is especially preferred.

No particular limitation is placed on the site where component (C) is present. Component (C) may be simply blended with components (A) and (B), or may be incorporated in component (A) or (B). It is especially preferable that component (C) is incorporated in component (A).

In the resin compositions for the formation of denture bases according to the present invention, no particular limitation is placed on the proportions of components (A), (B) and (C). However, component (B) is preferably used in an amount of 30 to 150 ml per 100 g of component (A). If the amount of component (B) is less than the aforesaid range, it becomes difficult to form denture bases. If the amount of component (B) is greater than the aforesaid range, polymerization shrinkage of the molded articles tends to be so marked that the resin composition lacks practical utility. Component (C) is preferably used in an amount of 0.1 to 5% by weight based on the weight of component (B). In order to impart sufficient impact resistance to denture bases, the graft copolymer is preferably used in an amount of 0.5 to 50% by weight, more preferably 1 to 30% by weight, based on the total weight of the resin composition.

The present invention is further illustrated by the following examples.

Reference Example 1 (Preparation of a rubber latex)

A solution composed of 4.0 kg of n-butyl acrylate, 20 g of diisopropylbenzene hydroperoxide, 100 g of beef tallow fatty acid potassium, 50 g of sodium N-lauroylsarcosinate, 50 g of sodium pyrophosphate, 0.5 g of ferrous sulfate, 30 g of dextrose and 20 kg of deionized water was deaerated by the passage of nitrogen and then charged into a 40-liter autoclave. After the addition of 6.0 kg of butadiene, the resulting mixture was polymerized at 50°C for 9 hours to obtain a rubber latex having a particle diameter of 0.07 μm.

Reference Example 2 (Preparation of a latex for particle enlargement)

A 5-liter round flask fitted with a stirrer was charged with a solution composed of 250 g of n-butyl acrylate, 20 g of potassium oleate, 10 g of sodium dioctylsulfosuccinate, 1.0 g of cumene hydroperoxide, 3 g of sodium formaldehyde sulfoxylate and 2,000 g of deionized water. After deaeration by the passage of nitrogen, this solution was polymerized at 70°C for 1.5 hours. Then, a solution composed of 600 g of n-butyl acrylate, 150 g of methacrylic acid and 3 g of cumene hydroperoxide was added dropwise thereto at 70°C over a period of an hour, and the stirring was continued at the same temperature for an additional hour to obtain a latex for particle enlargement.

Reference Example 3 (Preparation of a graft copolymer)

An autoclave having a capacity of 60 liters was charged with the rubber latex obtained in Reference Example 1, which was used in an amount containing 10 kg of polymer solids. While the rubber latex was being stirred, 1.5 kg of a 10% aqueous solution of sodium sulfate was added thereto. Then, the rubber latex was maintained at a bulk temperature of 50°C for 15 minutes and enlarged by adding thereto 152 g of the latex for particle enlargement obtained in Reference Example 2 and allowing the resulting mixture to stand for 30 minutes. Thereafter, the enlargement was stopped by adding a mixed solution containing, for each part of the polymer, 10 parts of water, 0.5 part of sodium sulfate and 0.5 part of sodium N-lauroylsarcosinate and allowing the resulting mixture to stand for 15 minutes. Thus, there was obtained an enlarged rubber latex having an average particle diameter of 0.148 μm.

To this enlarged rubber latex (containing 10 kg of polymer solids) were added 9 kg of deionized water, 20 g of sodium formaldehyde sulfoxylate and 50 g of sodium N-lauroylsarcosinate. After the resulting mixture was heated to 75°C, a mixed solution composed of 4,320 g of methyl methacrylate, 180 g of ethyl acrylate, 6.75 g of n-octyl mercaptan and 16 g of cumene hydroperoxide was added dropwise thereto over a period of 90 minutes. After completion of the addition, the resulting mixture was held at that temperature for an additional 60 minutes to obtain a graft copolymer latex L-1. To this graft copolymer latex L-1 were added 58 g of styrenated phenol, 44 g of dilauroyl thiodipropionate and 58 g of triphenyl phosphite. Then, using a 25% aqueous solution of sulfuric acid at a latex-solution ratio of 1:2, the latex was coagulated at 50°C and allowed to stand at 85°C for 5 minutes. The resulting polymer in slurry form was washed, dehydrated and dried at 55°C for 36 hours to obtain a white powder of graft copolymer G-1.

Reference Example 4

A rubber latex (having an average particle diameter of 0.07 μm) was prepared in the same manner as described in Reference Example 1, except that 5.0 kg of n-butyl acrylate, 5.0 kg of 1,3-butadiene, 120 g of beef tallow fatty acid potassium and 80 g of sodium N-lauroylsarcosinate were used. Then, this rubber latex was enlarged in the same manner as described in Reference Example 3 to obtain a latex having an average particle diameter of 0.148 μm. To this latex were added 5 kg of deionized water, 7 g of sodium formaldehyde sulfoxylate and 10 g of sodium N-lauroylsarcosinate. After the resulting mixture was heated to 80°C, a mixture comprising 25 kg of a mixed solution composed of 80% by weight of n-butyl acrylate, 18.2% by weight of styrene, 0.3% by weight of 1,4-butanediol diacrylate and 1.5% by weight of allyl cinnamate, and 7.5 g of butyl hydroperoxide was added dropwise thereto over a period of 170 minutes. Thereafter, the stirring was continued for an additional 180 minutes to obtain a composite rubber latex having an average particle diameter of 0.21 μm.

The above composite rubber latex was taken as a rubber latex in an mount corresponding to 12.5 kg of polymer solids, and 1 kg of deionized water, 20 g of sodium formaldehyde sulfoxylate and 50 g of sodium N-lauroylsarcosinate were added thereto. After the resulting mixture was heated to 75°C, a mixed solution of grafting monomers was added dropwise thereto and graft polymerization was carried out in the same manner as described in Reference Example 3. Thus, there was obtained a graft copolymer latex L-2.

Thereafter, this graft copolymer latex L-2 was treated in the same manner as described in Reference Example 3 to obtain a dry powder of graft copolymer G-2.

Reference Example 5

A 40-liter polymerization vessel was charged with a mixed solution composed of 70 g of sodium dialkylsulfosuccinate, 5 g of sodium carbonate, 80 g of potassium persulfate and 20 kg of deionized water. After this mixed solution was heated to 70°C, a mixed solution composed of 8.0 kg of n-butyl acrylate, 2.0 kg of styrene and 80 g of allyl methacrylate was added dropwise thereto over a period of 2 hours. Then, the stirring was continued at that temperature for an hour to obtain a rubber latex having an average particle diameter of 0.05 $\mu$m.

The rubber latex thus obtained was transferred to a 100-liter polymerization vessel. While this rubber latex was being stirred, 1.5 kg of a 10% aqueous solution of sodium sulfate and 0.1 kg of a 10% aqueous solution of sodium hydroxide were added thereto at a bulk temperature of 50°C. After the stirring was continued for 15 minutes, 152 g of the latex for particle enlargement obtained in Reference Example 2 was added and the resulting mixture was stirred for 30 minutes. Thereafter, the enlargement was stopped by adding a mixed solution containing, for each part of the polymer, 10 parts of water, 0.5 part of sodium sulfate and 0.5 part of sodium N-lauroylsarcosinate and allowing the resulting mixture to stand for 15 minutes. Thus, there was obtained a crosslinked rubbery copolymer latex having an average particle diameter of 0.19 $\mu$m.

To this latex was added an aqueous solution prepared by dissolving 20 g of sodium formaldehyde sulfoxylate, 50 g of sodium dialkylsulfosuccinate, 30 mg of ferrous sulfate and 90 mg of disodium ethylenediaminetetraacetate in deionized water. After the resulting mixture was heated to 75°C, a mixed solution composed of 4,320 g of methyl methacrylate, 180 g of ethyl acrylate, 6.75 g of n-octyl mercaptan and 16 g of cumene hydroperoxide was added dropwise thereto over a period of 90 minutes. After completion of the addition, the resulting mixture was held at that temperature for an additional 60 minutes to obtain a graft copolymer latex L-3.

Then, 10 kg of this latex was poured into an aqueous solution prepared by dissolving 500 g of magnesium sulfate in 20 kg of water. The resulting mixture was heated to 50°C and allowed to stand for 5 minutes. The resulting polymer in slurry form was washed, dehydrated and dried at 70°C for 24 hours to obtain a white dry powder of graft copolymer G-3.

Example 1 (Preparation of spherical particles J-1)

A 50-liter polymerization vessel was charged with 16 kg of deionized water and 80 g of polyvinyl alcohol (having a weight average molecular weight of 25,000 and a degree of saponification of 88%). While the contents were being stirred (at a speed of 200 rpm), the bulk temperature was raised to 60°C. A solution comprising graft copolymer G-1 highly dispersed in methyl methacrylate (which had been obtained by agitating and mixing 800 g of graft copolymer G-1, 32 g of benzoyl peroxide and 3.2 kg of methyl methacrylate in a homomixer) was added dropwise thereto over a period of 30 minutes. After completion of the addition, the resulting mixture was held at that temperature for an additional 200 minutes to effect suspension polymerization.

The resulting polymer was washed with water and dried at 80°C for 24 hours to obtain spherical particles J-1 having a particle diameter of 115 $\mu$m.

Examples 2 to 5 (Preparation of spherical particles J-2 to J-5)

Using various graft copolymers, benzoyl peroxide (BPO) and methyl methacrylate (MMA) in the respective amounts shown in Table 1, spherical particles J-2 to J-5 having particle diameters of 80 to 120 $\mu$m were prepared in the same manner as described in Example 1.

EP 0 584 376 A1

## Table 1

| Example | Graft copolymer Type | Amount (g) | Polymerization initiator Type | Amount (g) | Methacrylic ester Type | Amount (kg) | Spherical particles Type |
|---|---|---|---|---|---|---|---|
| 1 | G-1 | 800 | BPO | 32 | MMA | 3.2 | J-1 |
| 2 | " | 400 | " | 36 | " | 3.6 | J-2 |
| 3 | " | 200 | " | 38 | " | 3.8 | J-3 |
| 4 | G-2 | 400 | " | 36 | " | 3.6 | J-4 |
| 5 | G-3 | 400 | " | 36 | " | 3.6 | J-5 |

Example 6 (Preparation of spherical particles J-6)

Suspension polymerization was carried out under the same conditions as described in Example 1, except that the methyl methacrylate was replaced by a mixture composed of 3 kg of methyl methacrylate and 0.2 kg of benzyl methacrylate. Thus, there were obtained spherical particles J-6.

Example 7 (Preparation of spherical particles J-7)

Suspension polymerization was carried out under the same conditions as described in Example 1, except that the methyl methacrylate was replaced by a mixture composed of 3.1 kg of methyl methacrylate and 0.1 kg of phenyl methacrylate. Thus, there were obtained spherical particles J-7.

8

Example 8 (Preparation of spherical particles J-8)

Suspension polymerization was carried out under the same conditions as described in Example 1, except that the methyl methacrylate was replaced by a mixture composed of 3.1 kg of methyl methacrylate and 0.1 kg of ethylene glycol dimethacrylate. Thus, there were obtained spherical particles J-8.

Example 9 to 16

A rectangular negative mold formed of gypsum and measuring 20 mm x 15 mm x 3 mm was placed in the center of an FRP flask. The powder and liquid materials according to each of the formulations shown in Table 1 was blended together, and the resulting resin composition in the form of rice-cake was filled into the negative mold so as to leave no space therein. In this case, BPO was simply blended into the powder material. The flask was placed in a steam drier maintained at 70°C and the resin composition was prepolymerized for 30 minutes. Thereafter, the resin composition was polymerized for an hour in a steam drier maintained at 100°C. After the flask was air-cooled for 20 minutes, the molded piece was removed and cut into a specimen measuring 10 mm x 15 mm x 3 mm. Using this specimen, its impact strength (Dynstat impact strength) was measured. In addition, the workability of the resin composition was evaluated on the basis of the ease with which the powder and liquid materials were blended together and the resulting resin composition was filled into the negative mold. The resin composition was rated as good when it could be easily filled into the negative mold, and poor when it was difficult to fill the resin composition into the negative mold. The results thus obtained are shown in Table 2.

Table 2

| | Powder material | | | Liquid material (ml) | Impact strength (kg·cm/cm²) | Work-ability | Content of rubber in molded piece (%) |
|---|---|---|---|---|---|---|---|
| | Component (A) | | Component (C) | Component (B) | | | |
| | Spherical particles (g) | Graft copolymer in component (A) (wt.%) | BPO (g) | MMA | | | |
| Example 9 | J-1  60 | 20 | 1 | 40 | 22.8 | Good | 8.0 |
| " 10 | J-2  60 | 10 | 1 | 40 | 21.7 | Good | 4.0 |
| " 11 | J-3  60 | 5 | 1 | 40 | 21.9 | Good | 2.0 |
| " 12 | J-4  60 | 10 | 1 | 40 | 20.0 | Good | 5.2 |
| " 13 | J-5  60 | 10 | 1 | 40 | 21.5 | Good | 3.3 |
| " 14 | J-6  60 | 20 | 1 | 40 | 20.5 | Good | 4.0 |
| " 15 | J-7  60 | 20 | 1 | 40 | 19.7 | Good | 4.0 |
| " 16 | J-8  60 | 20 | 1 | 40 | 18.5 | Good | 4.0 |

Examples 17 to 30

Resin compositions were prepared and evaluated in the same manner as described in Example 9, except that each of various mixtures of MMA and ethylene glycol dimethacrylate (1G) or triethylene glycol dimethacrylate (3G) was used as the liquid material. The results thus obtained are shown in Table 3.

Table 3

| | Powder material | | Liquid material (ml) | | | Impact strength (kg·cm/cm²) | Work-ability |
|---|---|---|---|---|---|---|---|
| | Component (A) Spherical particles (g) | Component (C) BPO (g) | Component (B) | | | | |
| | | | MMA | 1G | 3G | | |
| Example 17 | J-1  60 | 1 | 36 | 4 | | 22.4 | Good |
| " 18 | J-1  60 | 1 | 36 | | 4 | 21.7 | Good |
| " 19 | J-2  60 | 1 | 36 | 4 | | 21.5 | Good |
| " 20 | J-2  60 | 1 | 36 | | 4 | 21.8 | Good |
| " 21 | J-3  60 | 1 | 38 | 2 | | 19.9 | Good |
| " 22 | J-3  60 | 1 | 38 | | 2 | 20.1 | Good |
| " 23 | J-4  60 | 1 | 36 | 4 | | 21.5 | Good |
| " 24 | J-4  60 | 1 | 36 | | 4 | 20.1 | Good |
| " 25 | J-5  60 | 1 | 38 | 2 | | 22.4 | Good |
| " 26 | J-5  60 | 1 | 38 | | 2 | 22.4 | Good |
| " 27 | J-6  60 | 1 | 36 | 4 | | 19.8 | Good |
| " 28 | J-6  60 | 1 | 36 | | 4 | 20.1 | Good |
| " 29 | J-8  60 | 1 | 36 | 4 | | 19.2 | Good |
| " 30 | J-8  60 | 1 | 36 | | 4 | 18.6 | Good |

Example 31 (Preparation of spherical particles H-1)

Graft copolymer latex L-1 obtained in Reference Example 3 was taken in an amount containing 1 kg of polymer solids and cooled to 30°C. While this latex was being stirred at a speed of 350 rpm, 10 g of a 1.0% aqueous solution of sulfuric acid was added to form a partial coagulum having high viscosity. When 9,000 g of methyl methacrylate (MMA) having 90 g of benzoyl peroxide (BPO) dissolved therein was added thereto and, thereafter, 25 kg of deionized water and 900 g of a 3% aqueous solution of a methyl methacrylate-potassium acrylate copolymer as a suspension stabilizer were added thereto, the system passed from the highly viscous state into a suspension having low viscosity. This suspension was thermally polymerized at 60°C for 3.0 hours. The resulting polymer was separated by filtration and then dried at 60°C for 24 hours to obtain white spherical particles H-1 comprising aggregates of fine particles. These particles had an average particle diameter of 100 $\mu$m.

Examples 32 and 33 (Preparation of spherical particles H-2 and H-3)

White spherical particles H-2 and H-3 comprising aggregates of fine particles were prepared under the same conditions as described in Example 31, except that each of graft copolymer latexes L-2 and L-3 was used. These two types of spherical particles had average particle diameters of 110 $\mu$m and 95 $\mu$m, respectively.

Example 34 (Preparation of spherical particles K-1)

After 20 g of graft copolymer G-1 obtained in Reference Example 3 and 100 g of PMMA particles (Acricon AC, manufactured by Mitsubishi Rayon Co., Ltd.; with an average particle diameter 100 $\mu$m) were dissolved in 1 kg of dichloromethane, the resulting solution was agitated in a homomixer to obtain a solution having the graft copolymer and the PMMA highly dispersed therein. This solution was charged into a 3-liter vessel; to which an aqueous solution formed by dissolving 10 g of polyvinyl alcohol (having a weight average molecular weight of 1,500 and a degree of saponification of 88%) in 1 kg of deionized water was added. The resulting mixture was warmed at 37°C for 24 hours with stirring (at a speed of 200 rpm) to evaporate the dichloromethane therefrom.

The polymer so formed was separated by filtration, washed with water, and then dried at 80°C for 24 hours to obtain spherical particles K-1 having a particle diameter of 95 $\mu$m.

Reference Example 6 (Preparation of ground particles F-1)

To 100 g of a partial polymer of methyl methacrylate (having a viscosity of 100 centipoises and a degree of polymerization of 10%) was added 20 g of graft copolymer G-1 obtained in Reference Example 3. This mixture was agitated in a homomixer for about an hour to disperse the graft copolymer uniformly. Then, 0.06 part of azobis(2,4-dimethylvaleronitrile) was dissolved therein as a polymerization initiator. The resulting material was freed of dissolved oxygen by exposure to reduced pressure and then poured into a cell formed of a gasket and two tempered glass plates so that the polymerization product would have a thickness of 3 mm. Polymerization was carried out at 82°C for 60 minutes under an atmosphere of warm water and at 130°C for 30 minutes under an atmosphere of air. The resulting polymerization product in sheet form was reduced to powder by means of a crusher (UC-210S Crusher, manufactured by Hourai Tekkojo K.K.). The resulting ground powder F-1 had an average particle diameter of 80 $\mu$m.

Reference Example 7 (Preparation of ground particles F-2)

3.6 kg of PMMA particles (Acricon AC, manufactured by Mitsubishi Rayon Co., Ltd.; with an average particle diameter 100 $\mu$m) were mixed with 400 g of graft copolymer G-1. This mixture was dried at 80°C for 24 hours and then melt-blended to obtain pellets comprising the graft copolymer highly dispersed in PMMA. The melt blending was carried out at melt temperatures (200-250°C) by means of a 25 mm$\phi$ vented extruder (Model VSE-25-24, manufactured by Osaka Seiki Kosaku K.K.).

The pellets so formed were reduced to powder by means of a crusher. Thus, there were obtained ground particles F-2 having an average particle diameter of 80 $\mu$m.

Examples 35 and 36 (Preparation of spherical particles K-2 and K-3)

Spherical particles K-2 and K-3 were prepared under the same conditions as described in Example 34, except that ground particles F-1 or F-2 were used in place of the graft copolymer and the PMMA particles. These two types of spherical particles had average particle diameters of 85 $\mu$m and 97 $\mu$m, respectively.

Example 37 (Preparation of spherical particles K-4)

Spherical particles K-4 (having an average particle diameters of 110 $\mu$m) were prepared under the same conditions as described in Example 34, except that 120 g of ground particles F-2 and 1.2 g of BPO were used in place of the graft copolymer and the PMMA particles. The BPO remaining in spherical particles K-4 was determined by reacting a sample with o-tolidine to develop a color and measuring its absorbance with a spectrophotometer (Model UV-120-02, manufactured by Shimadzu Corp.). When calculated from a calibration curve, the amount of residual BPO was 1.0% by weight based on the polymer.

Example 38 (Preparation of spherical particles K-5)

After 400 g of graft copolymer G-1 obtained in Reference Example 3 and 3.6 kg of PMMA particles (Acricon AC, manufactured by Mitsubishi Rayon Co., Ltd.; with an average particle diameter 100 $\mu$m) were dissolved in 50 kg of chloroform, the resulting solution was agitated in a homomixer to obtain a solution having the graft copolymer and the PMMA highly dispersed therein. This solution was treated with a spray dryer (Mobil-Minor Type Spray Dryer, manufactured by Nilo Atomizer Co.; with a flow rate of 80 kg/h and an internal temperature of 100°C) to evaporate the chloroform from the solution.

The resulting powder was dried at 80°C for 24 hours to obtain spherical particles K-5 having a particle diameter of 130 $\mu$m.

Examples 39 to 46

Resin compositions were prepared and evaluated in the same manner as described in Example 9, except that each of various types of particles H-1 to H-3 and K-1 to K-5 was used as the powder material. The results thus obtained are shown in Table 4. In Example 45, however, a molded piece was formed by polymerizing the resin composition with the aid of BPO incorporated in the spherical particles, instead of BPO simply blended into the powder material.

Comparative Examples 1 and 2

Resin compositions were prepared and evaluated in the same manner as described in Example 9, except that each of two types of ground particles F-1 and F-2 was used as the powder material. The results thus obtained are shown in Table 4.

13

Table 4

| | Powder material | | | Liquid material (ml) | Impact strength (kg·cm/cm²) | Work-ability | Content of rubber in molded piece (%) |
|---|---|---|---|---|---|---|---|
| | Component (A) Particles (g) | Graft copolymer in component (A) (wt.%) | Component (C) BPO (g) | Component (B) MMA | | | |
| Example 39 | H-1 60 | 10 | 1 | 40 | 19.1 | Good | 4.0 |
| " 40 | H-2 60 | 10 | 1 | 40 | 17.3 | Good | 5.2 |
| " 41 | H-3 60 | 10 | 1 | 40 | 19.6 | Good | 3.3 |
| " 42 | K-1 60 | 17 | 1 | 40 | 20.1 | Good | 6.7 |
| " 43 | K-2 60 | 17 | 1 | 40 | 20.1 | Good | 4.3 |
| " 44 | K-3 60 | 10 | 1 | 40 | 20.5 | Good | 5.5 |
| " 45 | K-4 60 | 10 | 0.6 | 40 | 19.1 | Good | 4.3 |
| " 46 | K-5 60 | 10 | 1 | 40 | 19.5 | Good | 4.0 |
| Comparative Example 1 | F-1 60 | 17 | 1 | 40 | 20.7 | Poor | 6.7 |
| " 2 | F-2 60 | 10 | 1 | 40 | 20.1 | Poor | 4.0 |

Comparative Examples 3 to 8

Resin compositions were prepared and evaluated in the same manner as described in Example 9, except that each of various blends of PMMA particles (Acricon AC, manufactured by Mitsubishi Rayon Co., Ltd.; with an average particle diameter 100 μm) and a graft copolymer was used in place of the spherical

14

particles. The results thus obtained are shown in Table 5.

Table 5

| | Powder material | | | Liquid material (ml) | Impact strength (kg·cm/cm²) | Work-ability | Content of rubber in molded piece (%) |
|---|---|---|---|---|---|---|---|
| | PMMA (g) | Graft copolymer (g) | BPO (g) | MMA | | | |
| Comparative Example 3 | 60 | | 1 | 40 | 4.5 | Good | 0 |
| " 4 | 57 | G-1 3 | 1 | 40 | 9.2 | Poor | 2.0 |
| " 5 | 54 | G-1 6 | 1 | 40 | 12.1 | Poor | 4.0 |
| " 6 | 48 | G-1 12 | 1 | 40 | 13.8 | Poor | 8.0 |
| " 7 | 54 | G-2 6 | 1 | 40 | 10.5 | Poor | 5.2 |
| " 8 | 54 | G-3 6 | 1 | 40 | 12.7 | Poor | 3.3 |

Comparative Examples 9 to 17

Using acrylonitrile-butadiene rubber (NBR), styrene-butadiene rubber (SBR) or silicone rubber (SIR) in place of a graft copolymer, resin compositions were prepared according to the formulations shown in Table

15

5. These resin compositions were evaluated in the same manner as described in Example 9. The results thus obtained are shown in Table 6.

Table 6

| | Powder material | | | Liquid material (ml) | | | Impact strength (kg·cm/cm²) | Work-ability | Content of rubber in molded piece (%) |
|---|---|---|---|---|---|---|---|---|---|
| | PMMA (g) | Rubber (g) | BPO (g) | MMA | 1G | 3G | | | |
| Comparative Example 9 | 57 | NBR 3 | 1 | 40 | | | 3.2 | Poor | 2.3 |
| " 10 | 54 | NBR 6 | 1 | 40 | | | 5.4 | Poor | 4.6 |
| " 11 | 48 | NBR 12 | 1 | 40 | | | 5.5 | Poor | 9.2 |
| " 12 | 54 | SBR 6 | 1 | 40 | | | 3.3 | Poor | 5.6 |
| " 13 | 48 | SBR 12 | 1 | 40 | | | 4.7 | Poor | 11.2 |
| " 14 | 54 | SIR 6 | 1 | 40 | | | 3.6 | Poor | 4.8 |
| " 15 | 48 | SIR 12 | 1 | 40 | | | 4.0 | Poor | 9.6 |
| " 16 | 54 | NBR 6 | 1 | 36 | 4 | | 3.5 | Poor | 4.6 |
| " 17 | 54 | NBR 6 | 1 | 36 | | 4 | 5.5 | Poor | 4.6 |

As described above, the spherical particles for use in denture base materials according to the present invention and the resin compositions containing them exhibit excellent moldability and, in particular, excellent workability at the time of molding. Moreover, molded articles formed therefrom have excellent impact resistance and are very useful as dental materials.

**Claims**

1.  Spherical particles (A) for use in denture base materials consisting of a graft copolymer (A-1) formed by the graft polymerization of a rigid resin component onto a crosslinked rubbery (co)polymer, and a methyl methacrylate (co)polymer (A-2).

2.  Spherical particles for use in denture base materials as claimed in claim 1 wherein the crosslinked rubbery (co)polymer comprises one or more members selected from the group consisting of butadiene (co)polymers, polyalkyl acrylate rubber copolymers, and composite rubbers formed by polymerizing a polyalkyl acrylate rubber copolymer in the presence of a butadiene polymer.

3.  Spherical particles for use in denture base materials as claimed in claim 2 wherein the cross linked rubbery (co)polymer has a number-average particle diameter of 0.1 to 1 $\mu$m.

4.  Spherical particles for use in denture base materials as claimed in claim 1 wherein the monomer used for the formation of the rigid resin component is styrene, methyl methacrylate, a mixture of these monomers, or a mixture of either of these monomers and a monomer copolymerizable therewith.

5.  Spherical particles for use in denture base materials as claimed in claim 1 wherein the copolymerizable monomer comprises one or more compounds selected from the group consisting of methyl acrylate, ethyl acrylate, phenyl methacrylate, benzyl methacrylate, isopropyl methacrylate and acrylonitrile.

6.  Spherical particles for use in denture base materials as claimed in claim 1 wherein the graft copolymer (A-1) is formed by the graft polymerization of 10 to 1,000 parts by weight of a monomer for the formation of the rigid resin component in the presence of 100 parts by weight, on a solid basis, of a latex of the crosslinked rubbery (co)polymer.

7.  Spherical particles for use in denture base materials as claimed in claim 1 wherein the methyl methacrylate (co)polymer (A-2) is a polymer formed from methyl methacrylate alone or a mixture of 50% by weight or more of methyl methacrylate and 50% by weight or less of at least one monomer copolymerizable therewith.

8.  Spherical particles (A) for use in denture base materials as claimed in claim 1 wherein the graft copolymer (A-1) comprises 3 to 60% by weight of the spherical particles (A).

9.  Spherical particles for use in denture base materials as claimed in claim 1 which have an average particle diameter of 1 to 500 $\mu$m.

10. A process for preparing spherical particles for use in denture base materials which comprises the steps of forming a graft copolymer by the graft polymerization of a rigid resin component onto a crosslinked rubbery (co)polymer, dispersing the graft copolymer in methyl methacrylate, a monomer mixture containing methyl methacrylate, or a partial polymer thereof, and subjecting the resulting mixture to suspension polymerization.

11. A process as claimed in claim 10 wherein, in the suspension polymerization step, polyvinyl alcohol is used as a suspension stabilizer.

12. A process for preparing spherical particles for use in denture base materials which comprises the steps of forming a graft copolymer by the graft polymerization of a rigid resin component onto a crosslinked rubbery (co)polymer, dissolving a methyl methacrylate (co)polymer and the graft copolymer in a solvent, dispersing the resulting solution in an aqueous or gaseous phase, and removing the solvent.

13. A process as claimed in claim 12 wherein the solvent comprises at least one compound selected from the group consisting of chloroform, dichloromethane, toluene, ethyl acetate and methyl methacrylate.

14. A resin composition for the formation of denture bases comprising spherical particles (A) consisting of a graft copolymer (A-1) formed by the graft polymerization of a rigid resin component onto a crosslinked rubbery (co)polymer, and a methyl methacrylate (co)polymer (A-2); a component (B) comprising one or more monofunctional methacrylic esters or a mixture of one or more monofunctional methacrylic esters and one or more multifunctional methacrylic esters; and a polymerization initiator (C).

15. A resin composition for the formation of denture bases as claimed in claim 14 wherein the component (B) is present in an amount of 30 to 150 ml per 100 g of the spherical particles (A), and the polymerization initiator (C) is present in an amount of 0.1 to 5 parts by weight per 100 parts by weight of the component (B).

16. A resin composition for the formation of denture bases as claimed in claim 14 wherein the polymerization initiator (C) is incorporated in the spherical particles (A).

## INTERNATIONAL SEARCH REPORT

| | |
|---|---|
| International application No. | |
| | PCT/JP93/00304 |

### A. CLASSIFICATION OF SUBJECT MATTER

Int. Cl$^5$   A61K6/00, 6/083, C08F2/44

According to International Patent Classification (IPC) or to both national classification and IPC

### B. FIELDS SEARCHED

Minimum documentation searched (classification system followed by classification symbols)

Int. Cl$^5$   A61K6/00, 6/083, C08F2/44

Documentation searched other than minimum documentation to the extent that such documents are included in the fields searched

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP, A, 55-149302 (Dentsply International Inc.), November 20, 1980 (20. 11. 80), & EP, A, 14515 | 1-16 |
| Y | JP, A, 57-203006 (Towa Giken K.K.), December 13, 1982 (13. 12. 82), & DE, A, 320099 & US, A, 4455911 | 14-16 |
| Y | JP, A, 60-173058 (Dentsply International Inc.), September 6, 1985 (06. 09. 85), & EP, A, 142747 & US, A, 4551486 | 1-13 |

| [X] | Further documents are listed in the continuation of Box C. | [ ] | See patent family annex. |
|---|---|---|---|

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| May 11, 1993 (11. 05. 93) | June 1, 1993 (01. 06. 93) |

| Name and mailing address of the ISA/ | Authorized officer |
|---|---|
| Japanese Patent Office | |
| Facsimile No. | Telephone No. |

Form PCT/ISA/210 (second sheet) (July 1992)